# EUROPEAN PATENT APPLICATION

(11) **EP 1 990 047 A1**
(43) Date of publication of application: **12.11.2008**
(21) Application number: 07290602.7
(22) Date of filing: 11.05.2007
(51) Int. Cl.: A61K 31/015, A61K 31/045, A61K 31/19, A61P 25/00

(54) **Use of a monoterpene to treat or prevent stress**

(71) Applicant: AISA THERAPEUTICS, 91058 Evry Cedex (FR)
(72) Inventor: D'Alessio, Patrizia, 75007 Paris (FR)
(74) Representative: Colombet, Alain André

(57) **Abstract**

The present invention relates to the use of a monoterpene for the prevention, alleviation or treatment of a pathological or non-pathological stress condition of an individual.

## Description

The invention relates to the use of a monoterpene to treat or prevent stress.

Stress can be defined as the sum of physical and mental responses to an unacceptable disparity between real or imagined personal experience and personal expectations. Mental responses to stress include adaptive stress, anxiety, and depression. However, if stress persists, associated to the inevitable process of aging, it may lead to various chronic diseases, going from infectious to inflammatory diseases, from cancer to degenerative and age-related diseases. For a long time, only "extreme" stress has been acknowledged to induce immune and vascular responses, such as infection or hypertension. Now we know that even "moderate" stress, independent from conventional risk factors (psycho-social stress, dietary stress, smoking,...), such as maternal separation, psychological and immobilization stresses, stressful life events in the middle aged and the ederly, academic stress (Zimecki *et al.,* 2005), can induce a potent alteration of healthy conditions and consequently shorten life quality and lifespan (Zimecki and Artym, 2004).

Farm animals become stressed, too. In domesticated food animals, stress can affect meat quality, milk production, and general health. But animal stress must often be intuited from clues such as lower-than-anticipated weights and increased illness. Transport of animals (cows, lambs, pigs, ...) to the slaughterhouse, for example, or to a competition (horses, dogs, chicken) are associated to a number of potentially stressing factors. These factors have a physical origin (truck moves, impacts by stability loss) and a psychological origin because every change in the common situation is susceptible to cause fear. Meat qualities in one case and performances in the second case are highly influenced by the psychological state of animals during the pre-slaughter / slaughter and pre-competition/ competition periods.

Laboratory procedures have also been studied to evaluate their impact on laboratory animals. Significant changes in physiologic parameters correlated with stress (e.g., serum or plasma concentrations of corticosterone, glucose, growth hormone or prolactin, heart rate, blood pressure, and behavior) were associated in multiple species with three procedures : handling (any non-invasive manipulation occurring as part of routine husbandry, including lifting an animal and cleaning or moving an animal's cage), blood collection, and orogastric gavage (Balcombe et al., 2004). Animals responded with rapid, pronounced, and statistically significant elevations in stress-related responses for each of the procedures, although handling elicited variable alterations in immune system responses. These findings indicate that laboratory routines are associated with stress, and that animals do not readily habituate to them. These data suggest that significant fear, stress, and possibly distress are predictable consequences of routine laboratory procedures, and that these phenomena have substantial scientific and human implications for the use of animals in laboratory research.

In humans, by decreasing normal resistance, stressful life conditions shorten the individual's lifespan and support the emergence of various diseases. Moreover, in humans, the outcome of stressful life conditions might be more complex than in animals at least because of the role of language, written or spoken, and belief (religion, tradition and other social containers that make stress tolerable or intolerable).

Terpenes are a large and varied class of hydrocarbons, produced primarily by a wide variety of plants, particularly conifers, though also by some insects such as swallowtail butterflies, which emit terpenes from their osmeterium. They are the major components of resin, and of turpentine produced from resin. The name "terpene" is derived from the word "turpentine". When terpenes are modified chemically, such as by oxidation or rearrangement of the carbon skeleton, the resulting compounds are generally referred to as terpenoids. Some authors will use the term terpene to include all terpenoids. Terpenes and terpenoids are the primary constituents of the essential oils of many types of plants and flowers. Essential oils are used widely as natural flavor additives for food, as fragrances in perfumery, in aromatherapy, and in traditional and alternative medicines. Synthetic variations and derivatives of natural terpenes and terpenoids also greatly expand the variety of aromas used in perfumery and flavors used in food additives.

Terpenes are derived biosynthetically from units of isoprene, which has the molecular formula C₅H₈. The basic molecular formulas of terpenes are multiples of that, (C₅H₈)ₙ where n is the number of linked isoprene units. Isoprene itself does not undergo the building process, but rather activated forms, isopentenyl pyrophosphate (IPP or also isopentenyl diphosphate) and dimethylallyl pyrophosphate (DMAPP or also dimethylallyl diphosphate), are the components in the biosynthetic pathway. As chains of isoprene units are built up, the resulting terpenes are classified sequentially by size as hemiterpenes, monoterpenes, sesquiterpenes, diterpenes, sesterterpenes, triterpenes, and tetraterpenes.

Monoterpenes consist of *two isoprene* units and have the molecular formula C₁₀H₁₆. Examples of monoterpenes are: geraniol and limonene. Monoterpenes occur in monocyclic, bicyclic, and acyclic forms and are either simple or modified hydrocarbons. They are a class of isoprenoid molecules derived from the anabolism of acetate by the mevalonic acid branch biosynthetic pathways of plants.
D-Limonene ((4R)-1-methyl-4-isopropenylcyclohex-1-ene), a major component of orange peel oil and the prototype of monoterpenes in carcinogenesis studies, is formed by the cyclization of the 10-carbon isoprene intermediate geranylpyrophosphate.

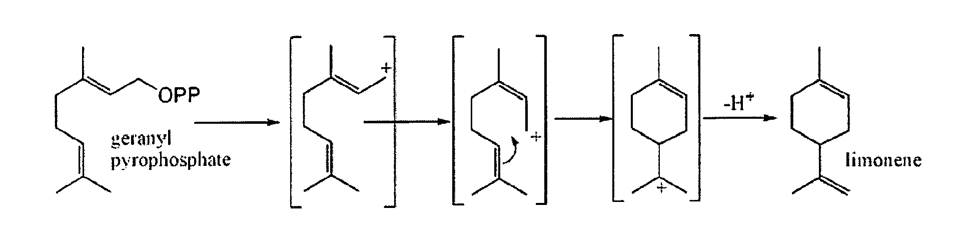

D-Limonene and its derived metabolites have been shown to possess cancer chemotherapeutic and chemopreventive efficacy in various preclinical model systems. Crowell PL et al. identified plasma metabolites of limonene in blood of seven healthy human volunteers having ingested 100 mg/kg limonene in a custard. On-line capillary gas chromatography/mass spectrometry analysis indicated that at least five compounds were present at 4 h after ingestion. Two major peaks were identified as the rat limonene metabolites dihydroperillic acid and perillic acid, and two minor peaks were found to be the respective methyl esters of these acids (Crowell PL et al., 1994).

Although R- and S-limonene are only weak inhibitors of the isoprenylation enzymes, their major metabolites, perillic acid and perillyl alcohol, are more potent inhibitors, with IC50 values in the low mM range. The metabolites possess greater activity towards the geranylgeranyltransferase type I enzyme than farnesyltransferase, while the novel metabolite displays IC50 values similar to those of perillic acid (Hardcastle IR et al., 1999).

D-Limonene has a pronounced chemotherapeutic activity and minimal toxicity in preclinical studies. A phase I clinical trial to assess toxicity, the maximum tolerated dose (MTD) and pharmacokinetics in patients with advanced cancer was followed by a limited phase II evaluation in breast cancer. D-Limonene is well tolerated in cancer patients at doses which may have clinical activity (Vigushin DM et al., 1998).

Unexpectedly, the present inventors have shown an anti-stress effect of limonene.

By a Functional Observation Battery (FOB, 44 parameters addressing behavioural, physiological and neurological parameters) in rats submitted to several stressful conditions (MacPhail, 1987), limonene and perillic alcohol have shown an important effect leading to the capacity to tolerate stress when compared to vehicle-treated animals.

Eighteen female rats per condition received orally 10 mg/kg of limonene, 10 mg/kg of perillic alcohol or a control carrier. 1, 2 or 3 hours later, a Functional Observation Battery (FOB) was carried out. The animals treated by limonene and perillic alcohol in comparison with the control showed :
**1/ Changes in the reaction to pain and stress**
   - an increase in interest for an object, curiosity
   - less quickly escapes after finger approach,
   - an increase in time before the rat falls,
   - a decrease in toe pinch reflex,
   - a high decrease in pain response,
   - no aggression,
   - no irritability,
   - very poor audible vocalization,
   - an important decrease in the startle response.
**2/ Changes in locomotive activity**
   - an increase of the number of squares crossed,
   - an increase in spontaneous activity,
   - an increase in wire manoeuvre
**3/ Physiological reactions**
   - a decrease of the body temperature,
   - an increase of the mictions

These results indicate an appeasing, relaxing effect, an anti-pain effect and an effect on temper giving a cheerful character of limonen and perillic alcohol with an increased physical condition.

Limonene and perillic alcohol show a rapid response in less than one hour.

Limonene shows an increasing effect during at least 3 hours whereas the effect of perillic alcohol has already decreased after 2 hours. This is consistent with the fact that a metabolite is assimilated faster than its original molecule.

### Use of a monoterpene for the prevention or alleviation of a stress condition

Thus, the present invention relates to the use of a monoterpene for the prevention, alleviation or treatment of a stress condition of an individual. A "stress condition" means the condition of an individual in response to stressing conditions.

Stress conditions comprise pathological and non-pathological stress conditions.

In the context of the invention, a "pathological stress condition" means an episodic acute stress condition or a chronic stress condition.

"Episodic acute stress" means a frequently acute stress. People who endure acute stress frequently always seem to be facing multiple stressful situations. They're always in a rush, always late, always taking on too many projects, handling too many demands. Unlike people for whom stress is a once-in-a-while spike, these folks are experiencing episodic acute stress.

"Chronic stress" means herein unrelenting demands and pressures for seemingly interminable periods of time. Chronic stress is stress that wears you down day after day and year after year, with no visible escape. It grinds away at both mental and physical health, leading to breakdown and even death and altogether to premature aging.

In the context of the invention, a "non-pathological stress condition" means "acute stress", i.e. a punctual stress condition in response to a change in the familiar environment. Acute stress is the most common and most recognizable form of stress, the kind in which you know exactly why you're stressed: you were just in a car accident; the school nurse just called; a bear just ambled onto your campsite. Or it can be something scary but thrilling, such as a parachute jump. Along with obvious dangers and threats, common causes of acute stressors include noise, isolation, crowding, and hunger. Normally, your body rests when these types of stressful events cease and your life gets back to normal.

As used herein, the term "individual" denotes an animal or a human, preferably a bird or a mammal, such as a rodent, a feline, an equine, a bovine, a caprine, a canine, a porcine and a primate. Preferably, an individual according to the invention is a human. Preferably, an individual according to the invention is a cat or a dog.

In the context of the invention, the terms "to prevent", "preventing" or "prevention" means to allow avoiding a stress condition.

In the context of the invention, the terms "to alleviate", "alleviating" or "alleviation" means to allow decreasing one or more stress symptoms.

In the context of the invention, the terms "to treat", "treating" or "treatment", means reversing, alleviating, or inhibiting the course of a pathological stress condition or one or more symptoms thereof.

Intellectual symptoms of stress include: problems with memory, difficulty making decisions, inability to concentrate, shortened attention span, confusion, repetitive or continual thoughts, misunderstanding of what others tell you, poor judgment, thoughts of escaping, running away, inability to slow down thought process, and/or loss of objectivity.

Physical symptoms of stress include: headaches, digestive disorders, muscle tension and pain, sleep disturbances, fatigue, chest pain, irregular heartbeat, high blood pressure, weight gain or loss, hair loss, asthma or shortness of breath, skin problems, periodontal disease, jaw pain, reproductive problems, such as missed periods, immune system suppression, and/or sweatiness.

Emotional symptoms of stress include: less interest in hobbies or fun, sudden shifts in mood, frequent uneasiness, restlessness, frustration, anger, resentment, unwarranted jealousy, quick irritability with others, oversensitivity, overreaction to unexpected situations or events, sense of being overwhelmed or swamped, anxiety, increased fear of failure, inadequacy, reduced confidence, depression, apathy, and/or desire to cry.

Behavioral symptoms of stress include: eat more or less, sleep too much or too little, isolate yourself from others, including people close to you, stay home from work or stay at work extended hours, increase use of tobacco, alcohol, drugs, caffeine, have sex more or less, engage in nervous habits such as nail biting, hair twisting, pacing, grind your teeth, laugh or cry at inappropriate times, overdo activities such as exercising or shopping, become bossy or inflexible with others, lose your temper, argue with people, become violent, take inappropriate risks, and/or exhibit road rage.

In the frame of the use of a monoterpene according to the invention, the monoterpene may be administered by a systemic way, preferably by the oral way.

### Pathological stress conditions

A further object of the present invention relates to the use of a monoterpene for the manufacture of a medicament for the prevention, alleviation or treatment of a pathological stress condition of an individual.

In a preferred embodiment, said prevention, alleviation or treatment concerns a human, a pet, a laboratory animal or a farm animal. More preferably, said prevention, alleviation or treatment concerns an individual selected in the group consisting of a bird or a mammal, such as a rodent, a feline, an equine, a bovine, a caprine, a canine, a porcine and a primate. Most preferably, said prevention, alleviation or treatment concerns a human, a cat or a dog.

Preferably, the symptoms of said pathological stress condition are anxiety, depression, problems with memory, inability to concentrate, sleep alterations, insomnia or exaggerated sleep, troubled sleep, eating problems, either bulimia or anorexia, with no respect of feeding rhythms, recurrence to drugs, comprehensive of cigarette smoking, chronic fatigue, demotivation, resignation, and/or any symptom able to reduce the performance of an individual having no detectable pathology.

For animals this implies also sleep alterations, insomnia or exaggerated sleep, troubled sleep, eating problems, either bulimia or anorexia, with no respect of feeding rhythms, chronic fatigue, resignation, depression and/or any symptom deteriorating the quality of the meat in the case of slaughter animals having no detectable pathology, but also animal derived products such as eggs, milk or other products such as wool or feathers, and of performance in the case of competition animals.

Such medicaments comprise a monoterpene as active ingredient and pharmaceutically acceptable carriers.

"Pharmaceutically acceptable" refer to molecular entities and compositions that do not produce an adverse, allergic or other untoward reaction when administered to an animal, or a human, as appropriate.

As used herein, "pharmaceutically acceptable carrier" includes any and all solvents, dispersion media, coatings, antibacterial and antifungal agents, isotonic and absorption delaying agents and the like. The use of such media and agents for pharmaceutical active substances is well known in the art. Except insofar as any conventional media or agent is incompatible with the monoterpene used according to the invention, its use in the medicaments, or for implementing the methods for preventing or treating a pathological stress condition in an individual according to the invention is contemplated. Supplementary active ingredients can also be incorporated into the medicaments.

In a further embodiment, the invention relates to a method for preventing, alleviating or treating a pathological stress condition in an individual.

Preferably, a method for preventing, alleviating or treating a pathological stress condition in an individual according to the invention comprises the administration of a medicament comprising a monoterpene, in one dose or in repeated doses and at specified intervals of time.

In the frame of methods for preventing, alleviating or treating a pathological stress condition in an individual according to the invention, the administration regimen may be orally.

In the frame of methods for preventing, alleviating or treating a pathological stress condition in an individual according to the invention, the administration regimen may be for instance for a period of more than 4 weeks, or more than 8 weeks. The molecule not being toxic the treatment can be continued until the patient resents its benefits i.e. for years or his whole life-time. This is particularly valid for those populations who are at risk to develop stress, so that they would take the molecules as stress prevention.

Preferably, in the frame of methods for preventing, alleviating or treating a pathological stress condition in an individual according to the invention, the range of dose of monoterpene may be between 0.1 mg/kg to 100 mg/kg/day. More preferably, the dose range is between 1 mg to 100 mg/kg. Most preferably, the dose range is between 10 mg to 50 mg/kg. More preferably, the dose range is between 10 mg/kg/day to 50 mg/kg/day.

Preferably, the use of a monoterpene for the prevention, alleviation or treatment of a pathological stress condition of an individual may be by oral route, mucosal route or cutaneous route. As such the use of a monoterpene for the prevention, alleviation or treatment of a pathological stress condition of an individual is preferably in a way adapted for such administration routes.

Preferably, the use of a monoterpene for the prevention, alleviation or treatment of a pathological stress condition of an individual may be in a form selected in the group consisting of food complement, patch, capsule, pill, beverage and biscuit.

### Non-pathological stress conditions

A further object of the present invention relates to the use of a monoterpene for the prevention, alleviation or treatment of a non-pathological stress condition of an individual.

In a preferred embodiment, said prevention, alleviation or treatment concerns a human, a pet, a laboratory animal or a farm animal. More preferably, said prevention, alleviation or treatment concerns an individual selected in the group consisting of a bird or a mammal, such as a rodent, a feline, an equine, a bovine, a caprine, a canine, a porcine and a primate. Most preferably, said prevention, alleviation or treatment concerns a human, a cat or a dog.

Preferably, said non-pathological stress condition is due to force-feeding in ducks or geese or bovine or horses or sheep or chickens. More preferably, said non-pathological stress condition is due to force-feeding in ducks or geese.

Preferably, said non-pathological stress condition is due to carriage in animals transported for slaughter (ducks or geese or bovine or horses or sheep or chickens) or competition (horses or cats or dogs) purposes. More preferably, said non-pathological stress condition is due to carriage in horses.

Preferably, said non-pathological stress condition is due to a performance stress at school, theatre, sport, more preferably is due to an exam in human (called "academic stress").

In a further embodiment, the invention relates to a method for preventing, alleviating or treating a non-pathological stress condition of an individual.

Preferably, a method for preventing, alleviating or treating a non-pathological stress condition of an individual according to the invention comprises the administration of a food complement comprising a monoterpene, in one dose or in repeated doses and at specified intervals of time.

In the frame of methods for preventing, alleviating or treating a non-pathological stress condition of an individual according to the invention, the administration regimen may be for instance for a period of more than 4 weeks, or more than 8 weeks. As the molecule is not toxic under no circumstances at the preferential dose indicated, the administration period can extend to years if the individual resents benefits from the treatment.

Preferably, in the frame of methods for preventing, alleviating or treating a non-pathological stress condition of an individual according to the invention, the range of dose of monoterpene may be between 0.1 mg/kg to 100 mg/kg/day. More preferably, the dose range is between 1 mg to 100 mg/kg. Most preferably, the dose range is between 10 mg/kg to 50 mg/kg.

Preferably, the use of a monoterpene for the prevention, alleviation or treatment of a non-pathological stress condition of an individual may be by oral route, mucosal route or cutaneous route. As such the use of a monoterpene for the prevention, alleviation or treatment of a non-pathological stress condition of an individual is preferably in a way adapted for such administration routes.

Preferably, the use of a monoterpene for the prevention, alleviation or treatment of a non-pathological stress condition of an individual may be in a form selected in the group consisting of food complement, patch, capsule, pill, beverage and biscuit.

### Monoterpenes

Monoterpenes used in the present invention include limonene, geraniol, geranyl acetate, isomenthone, perillyl alcohol, perillic acid, dihydroperillic acid, the respective methyl esters of these acids, carvone, citral, menthol.

In the context of the invention, limonene means (R)-(+)-Limonen, i.e. D-limonene.

More preferably, monoterpenes used in the present invention are limonene, one of its metabolites or a mixture thereof.

Limonene metabolites include perillyl alcohol, perillic acid, dihydroperillic acid, and their respective methyl esters.

The invention will be further illustrated in view of the following figures and examples.

### FIGURES

Figure 1: is a graph showing the average of the body temperature of the animals 60 minutes before and 60, 120, 180 minutes after treatment for the three control, Limonen 10 and Perillic alcohol 10 groups.

Figure 2: is a graph showing the average of the number of squares crossed by the animals 60 minutes before and 60, 120, 180 minutes after treatment for the three control, Limonen 10 and Perillic alcohol 10 groups.

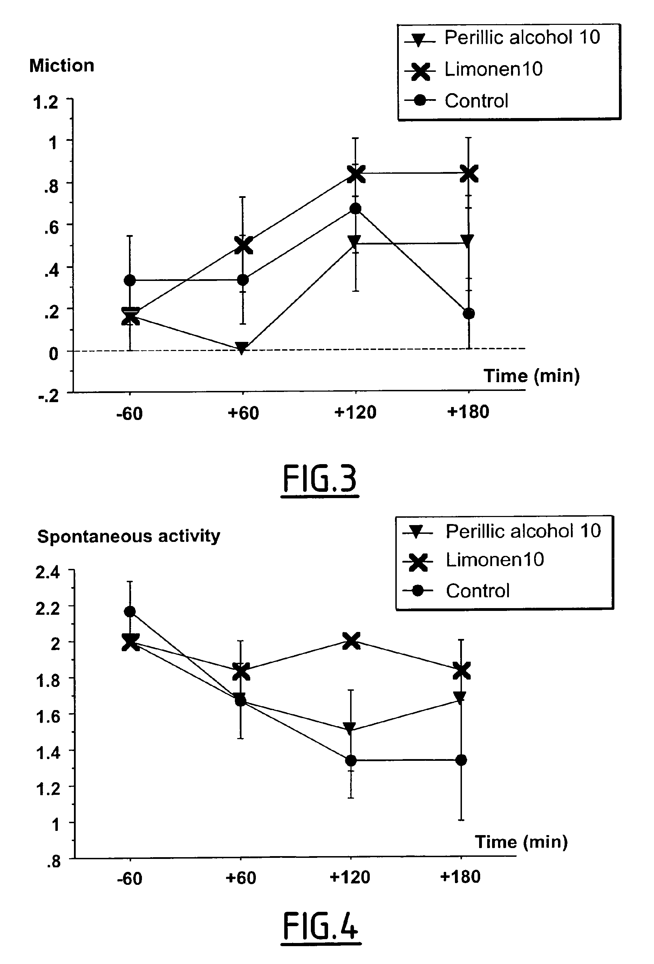

Figure 3: is a graph showing the average of the scores of mictions of the animals 60 minutes before and 60, 120, 180 minutes after treatment for the three control, Limonen 10 and Perillic alcohol 10 groups. Legend : 0 = no; 1 = yes

Figure 4: is a graph showing the average of the scores for the spontaneous activity test of the animals 60 minutes before and 60, 120, 180 minutes after treatment for the three control, Limonen 10 and Perillic alcohol 10 groups. Legend : 0 = nothing; 1 = small moves ; 2= moderate moves ; 3 = fast moves ; 4 = excited.

Figure 5: is a graph showing the average of the number of squares crossed on one way of the animals 60 minutes before and 60, 120, 180 minutes after treatment for the three control, Limonen 10 and Perillic alcohol 10 groups.

Figure 6: is a graph showing the average of the scores for the object interest test of the animals 60 minutes before and 60, 120, 180 minutes after treatment for the three control, Limonen 10 and Perillic alcohol 10 groups. Legend : 0 = no; 1 = look at ; 2 = follow.

Figure 7: is a graph showing the average of the scores for the finger approach test of the animals 60 minutes before and 60, 120, 180 minutes after treatment for the three control, Limonen 10 and Perillic alcohol 10 groups. Legend : 0 = no; 1 = small escape ; 2 = moderate escape ; 3 = vigorous escape.

Figure 8: is a graph showing the average of the scores for the wire manoeuver test of the animals 60 minutes before and 60, 120, 180 minutes after treatment for the three control, Limonen 10 and Perillic alcohol 10 groups. Legend : 0 = fall; 1 = fall after some seconds ; 2 = no success ; 3 =laboured success ; 4 = success.

Figure 9: is a graph showing the average of the scores for the negative geotaxis test of the animals 60 minutes before and 60, 120, 180 minutes after treatment for the three control, Limonen 10 and Perillic alcohol 10 groups. Legend : 0 = fall; 1 = no move; 2 = move but no look back ; 3 =look back and no further move ; 4 = look back and climb.

Figure 10: is a graph showing the average of the scores for the toe pinch test of the animals 60 minutes before and 60, 120, 180 minutes after treatment for the three control, Limonen 10 and Perillic alcohol 10 groups. Legend : 0 = light; 1 = moderate ; 2 = fast ; 3 = repeted.

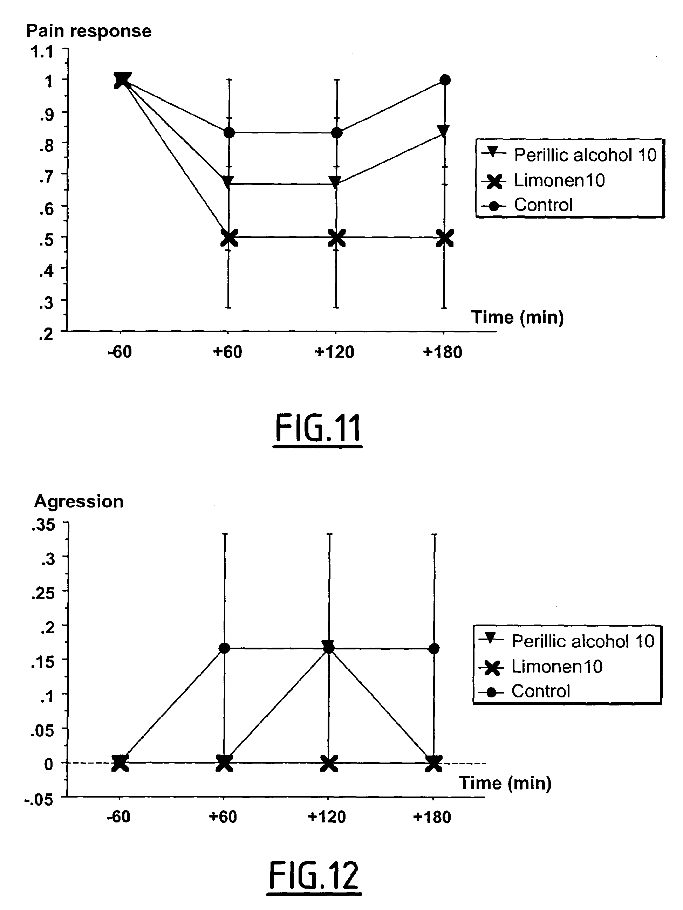

Figure 11: is a graph showing the average of the scores for the pain response test of the animals 60 minutes before and 60, 120, 180 minutes after treatment for the three control, Limonen 10 and Perillic alcohol 10 groups. Legend : 0 = no; 1 = yes.

Figure 12: is a graph showing the average of the scores for the agression test of the animals 60 minutes before and 60, 120, 180 minutes after treatment for the three control, Limonen 10 and Perillic alcohol 10 groups. Legend : 0 = no; 1 = yes.

Figure 13: is a graph showing the average of the scores for the irritability test of the animals 60 minutes before and 60, 120, 180 minutes after treatment for the three control, Limonen 10 and Perillic alcohol 10 groups. Legend : 0 = no; 1 = yes.

Figure 14: is a graph showing the average of the scores for the audible vocalization test of the animals 60 minutes before and 60, 120, 180 minutes after treatment for the three control, Limonen 10 and Perillic alcohol 10 groups. Legend : 0 = no; 1 = yes.

Figure 15: is a graph showing the average of the scores for the startle test of the animals 60 minutes before and 60, 120, 180 minutes after treatment for the three control, Limonen 10 and Perillic alcohol 10 groups. Legend : 0 = no; 1 = yes.

### Examples

### 1- MATERIAL AND METHODS

### 1.1 - Animals

Eighteen female rats Wistar HsdBrlHan EOPS (Ganat, France) with an average weight of 175-200 g are used.

The 18 rats are weighed, marked and shared in 3 groups of 6 (n = 6) :
- "control" group = oral treatment with maize oil ;
- "Limonene 10" group = oral treatment with Limonene at 10 mg/kg ;
- "Perillic alcohol 10" group = oral treatment with perillic alcohol at 10 mg/kg ;

### 1.2 - Assayed products

Limonene ((R)-(+)-Limonene, MW = 136.23, purity = 97%) from SIGMA-ALDRICH (Saint-Quentin Fallavier, France) has been used and stored according to SIGMA-FLUKA instructions.

Perillic alcohol ((S)-4-Isopropenyl-1-cyclohexenylmethanol; (S)-p-Mentha-1,8-dien-7-ol, MW = 152,23, purity = 98%) from SIGMA-ALDRICH (Saint-Quentin Fallavier, France) has been used and stored according to SIGMA-FLUKA instructions.

Limonene and perillic alcohol have been prepared in maize oil extemporarily every day of treatment at 4 mg/ml and administered par orally at 10 mg/kg for a volume of 2.5 ml/kg, just after the first assay at t = 0.

**Table 1 Summary of the treatment conditions**

| **Groups** | **rats Nr** | **Assayed product** | **Dosis (mg/kg)** | **FOB (minutes after administration)** |
|---|---|---|---|---|
| Control | 6 | Maize oil | - | 0, +60, +120, +180 |
| Limonene 10 | 6 | Limonene | 10 | |
| Perillic alcohol 10 | 6 | Perillic alcohol | 10 | |

### 1.3 - Effects of limonene or perillic alcohol on the Functional Observation Battery (FOB)

The observations are carried out 60 minutes before treatment and 60, 120 and 180 minutes after the administration orally of the assayed products and comprise three observation phases :
- a first direct observation phase during which the animal is not disturbed ;
- an active observation phase during which the animal is manipulated ;
- a phase dedicated to the evaluation of the responses of the animals towards the reactivity assays.
The studied variables are the following :
- behavioural effects : spontaneous locomotive activity, locomotive behaviour troubles, anxiety, touch response, irritability, aggression and freezing caused behaviours, somnolence, number of defecations, number of mictions, sensor-motor responses (toe pinch response and sound response) ;
- neurological effects: pupillary reflex, palpebral closure, pelvic elevation, tail position, limb and abdominal tones, reversal test, grip test, tremors and piloerection ;
- physiological effects: salivation, lacrimation, diarrhea, body temperature, respiratory rhythm.

The animals are weighed on the day of the administration of the assayed products.
During the experimentation, the animals are killed under anesthesia and autopsied if one of the following signs is observed :
- pain (cachexia, weakening, ...) ;
- products toxicity (abnormal behavior, convulsions, ...) ;
- weight loss of at least 25% through three consecutive days or 20% in one day.

### 1.4 - Statistics

Considering the Gaussian distribution or not of the data, parametric (P) or non-parametric (NP) tests are used : ANOVA (P) or Kruskal-Wallis test (NP) followed possibly by the Dunnett test (P) or the Mann-Whitney test (NP) to compare the treated groups with the control group. Statistics are carried out with the Statview 5 software (SAS, Institute Inc., USA).

### 2- RESULTS

### 2.1 - 60 minutes before treatment

At T-60, the Kruskal-Wallis test does not show any significant heterogeneity between the different groups for all the studied variables.

### 2.2 - 60 minutes after treatment (Table 2)

At T+60, the Kruskal-Wallis test shows significant heterogeneities between the different groups for the following variables :
- Interest for an object : H_{(ddl = 2)} = 9.316 ; p < 0.01 ;
- Toe pinch reflex : H_{(ddl = 2)} = 6.591 ; p < 0.05 ;
- Irritability : H_{(ddl = 2)} = 11.900 ; P < 0.01 ;
- Audible vocalization : H_{(ddl = 2)} = 8.075 ; p < 0.05.

The Kruskal-Wallis test shows heterogeneity tendencies between the different groups for the following variables :
- Wire manoeuver : H(_{ddl=2}) = 5.360 ; p < 0.10 ;
- Startles : H(_{ddl=2}) = 5.377 ; p < 0.10.

The Kruskal-Wallis test does not show any significant heterogeneity between the different groups for the other studied variables.
• The Mann-Whitney test is carried out to provide comparisons from one group to another.

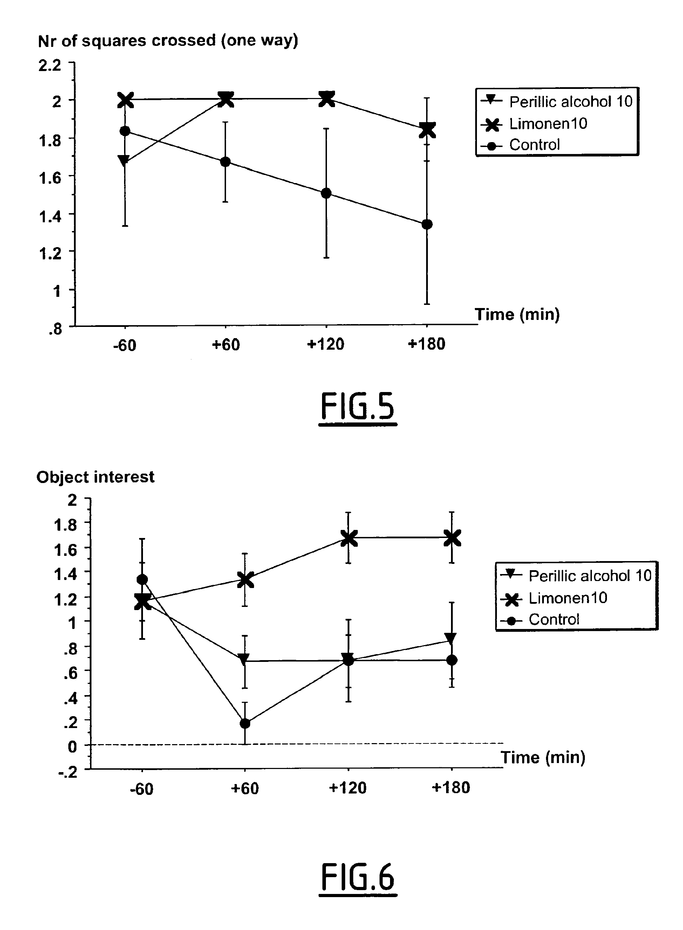

### Interest for an object (see Figure 6)

The Mann-Whitney test shows that the rats of the group Limonene 10 have an interest significantly more important for an object than those of the control group.
The Mann-Whitney test shows that the rats of perillic alcohol 10 group have a tendency to have a more important interest for an object than those of the control group.

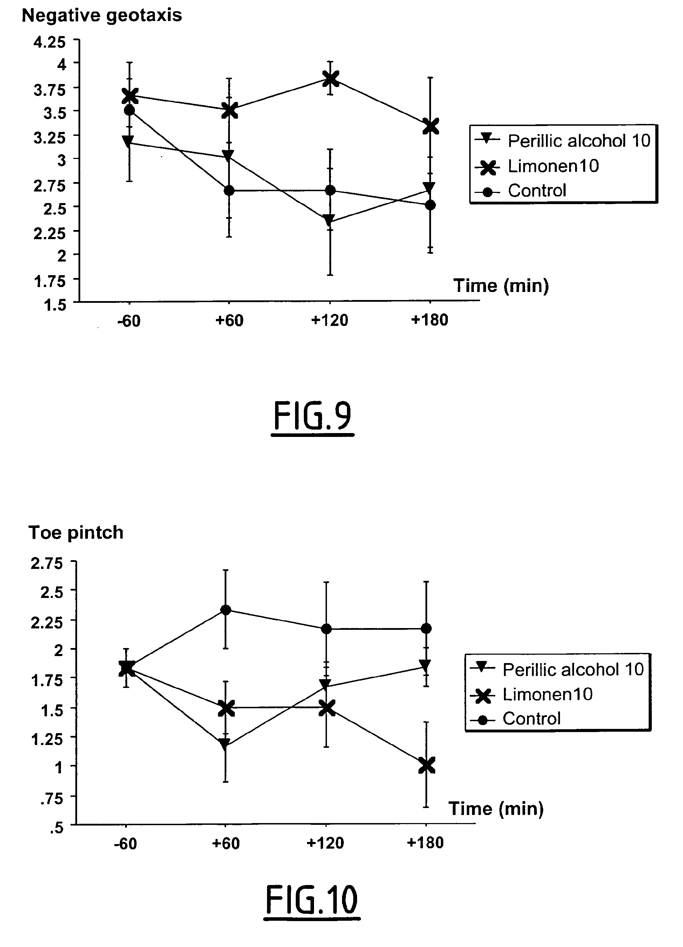

### Toe pinch reflex (see Figure 10)

The Mann-Whitney test shows that the rats of the Limonene 10 and perillic alcohol 10 groups are significantly less reactive of toe pinch than those of the control group.

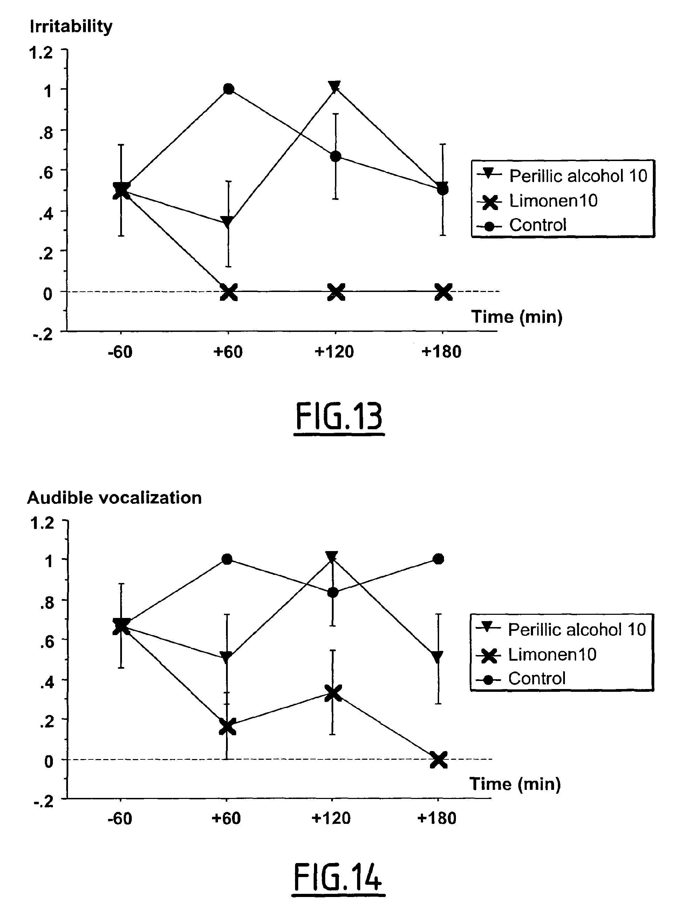

### Irritability (see Figure 13)

The Mann-Whitney test shows that the rats of the Limonene 10 and perillic alcohol 10 groups are significantly less irritable than those of the control group.

### Audible vocalization (see Figure 14)

The Mann-Whitney test shows that the rats of the Limonene 10 group emit significantly less vocalization than those of the control group.
The Mann-Whitney test shows that the rats of the perillic alcohol 10 group have a tendancy to emit less vocalization than those of the control group.

**Table 2 FOB results 60 minutes after treatment (T+60) Kruskal-Wallis test and Mann-Whitney test vs. Control**

| **Groups** | **Control** | **Limonene 10** | **Perillic alcohol 10** |
|---|---|---|---|
| **Variables** | **(n = 6)** | **(n = 6)** | **(n = 6)** |
| **Interest for an object** | | **>** | > |
| | | **p < 0.01** | p < 0.10 |
| **Toe pinch reflex** | | **<** | **<** |
| | | **p < 0.05** | **p < 0.05** |
| **Irritability** | | **<** | **<** |
| | | **p < 0.01** | **p < 0.05** |
| **Audible vocalization** | | **<** | < |
| | | **p < 0.01** | p < 0.10 |

| | | | |
|---|---|---|---|
| Legend of Table 2 : « > » means a reflex higher than the control group "<" means a reflex lower than the control group boldface symbols mean a significant effect | | | |

### 4.1.2.3 - FOB results 120 minutes after treatment (Table 3)

At T+120, the Kruskal-Wallis test shows significant heterogeneities between the different groups for the following variables :
- Interest for an object : H_{(ddl = 2)} = 6.800 ; p < 0.05 ;
- Finger approach : H_{(ddl = 2)} = 9.917 ; p < 0.01 ;
- Irritability : H_{(ddl = 2)} = 11.900 ; p < 0.01 ;
- Audible vocalization : H_{(ddl = 2)} = 6.800 ; p < 0.05 ;
- Startles : H_{(ddl = 2)} = 7.200 ; p < 0.05.

The Kruskal-Wallis test shows heterogeneity tendencies between the different groups for the following variables :
- Body temperature : H_{(ddl=2)} = 5.673 ; p < 0.10 ;
- Spontaneous activity : H_{(ddl=2)} = 5.740 ; p < 0.10 ;
- Negative geotaxis : H_{(ddl=2)} = 5.699 ; p < 0.10.

The Kruskal-Wallis test does not show any significant heterogeneity between the different groups for the other studied variables.
• The Mann-Whitney test is carried out to provide comparisons from one group to another.

### Interest for an object (see Figure 6)

The Mann-Whitney test shows that the rats of the group Limonene 10 have an interest significantly more important for an object than those of the control group.

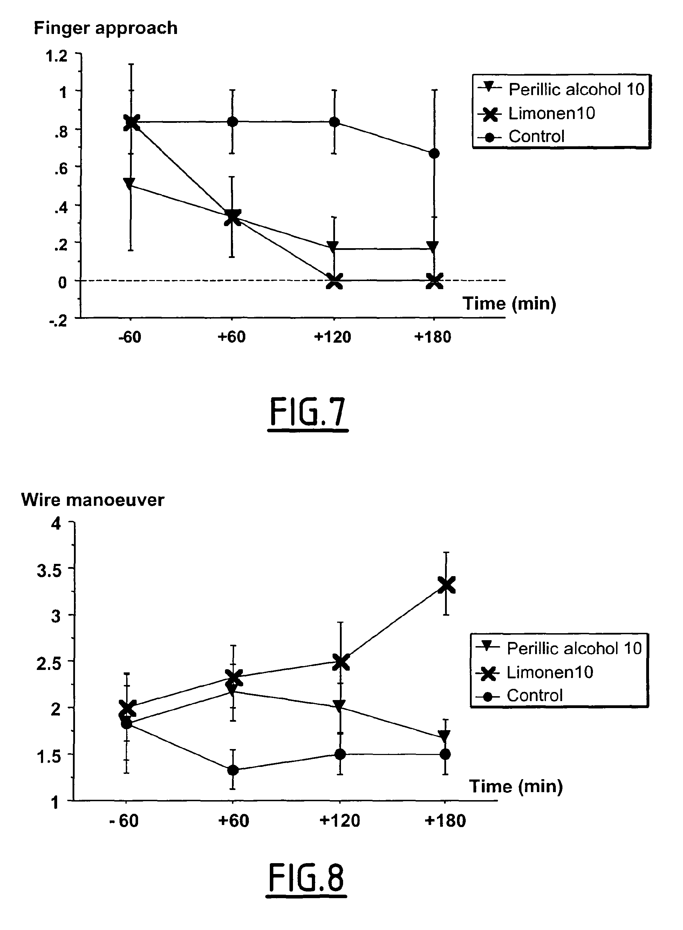

### Finger approach (see Figure 7)

The Mann-Whitney test shows that the rats of the Limonene 10 and Perillic alcohol 10 groups have significantly less escapes after finger approach than those of the control group.

### Irritability (see Figure 13)

The Mann-Whitney test shows that the rats of the Limonene 10 group are significantly less irritable than those of the control group.

### Audible vocalization (see Figure 14)

The Mann-Whitney test shows that the rats of the Limonene 10 group emit significantly less vocalization than those of the control group.

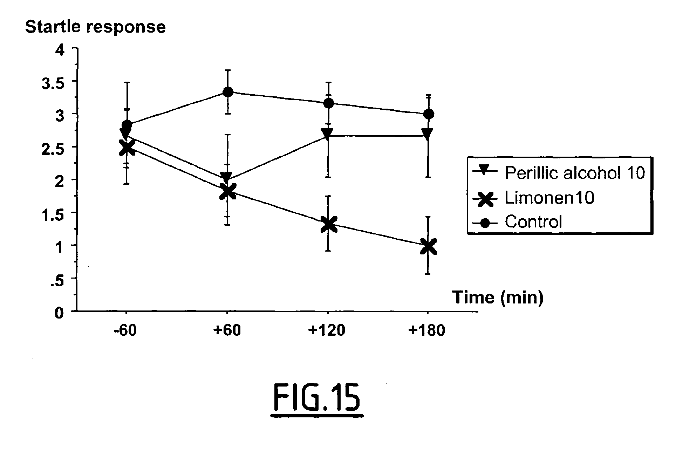

### Startles(see Figure 15)

The Mann-Whitney test shows that the rats of the Limonene 10 group have significantly less startles than those of the control group.

**Table 3 FOB results 120 minutes after treatment (T+120) Kruskal-Wallis test and Mann-Whitney test vs. Control**

| **Groups** | **Control** | **Limonene 10** | **Perillic alcohol 10** |
|---|---|---|---|
| **Variables** | **(n = 6)** | **(n = 6)** | **(n = 6)** |
| **Interest for an object** | | **>** | N.S. |
| | | **p < 0.05** | |
| **Finger approach** | | **<** | **<** |
| | | **p < 0.01** | **p < 0.05** |
| **Irritability** | | **<** | N.S. |
| | | **p < 0.05** | |
| **Audible vocalization** | | **<** | N.S. |
| | | **p < 0.05** | |
| **Startles** | | **<** | N.S. |
| | | **p < 0.01** | |

| | | | |
|---|---|---|---|
| Legend of Table 3 : ">" means a reflex higher than the control group "<" means a reflex lower than the control group boldface symbols mean a significant effect "N.S." = no difference with the control group | | | |

### 4.1.2.4 - FOB results 180 minutes after treatment (Table 4)

• At T+180, the Kruskal-Wallis test shows significant heterogeneities between the different groups for the following variables :
   - Body temperature: H_{(ddl = 2)} = 7.616 ; p < 0.05 ;
   - Number of squares crossed : H_{(ddl = 2)} = 6.125 ; p < 0.05 ;
   - Interest for an object: H_{(ddl = 2)} = 6.656 ; p < 0.05 ;
   - Wire manoeuver: H_{(ddl = 2)} = 10.579 ; p < 0.01 ;
   - Audible vocalization: H_{(ddl = 2)} = 11.333 ; p < 0.01 ;
   - Startles: H_{(ddl = 2)} = 7.766 ; p < 0.05.

The Kruskal-Wallis test shows heterogeneity tendencies between the different groups for the following variables :
- Miction : H_{(ddl=2)} = 5.037 ; p < 0.10 ;
- Toe pinch : H_{(ddl=2)} = 5.217 ; p < 0.10.

The Kruskal-Wallis test does not show any significant heterogeneity between the different groups for the other studied variables.
• The Mann-Whitney test is carried out to provide comparisons from one group to another.

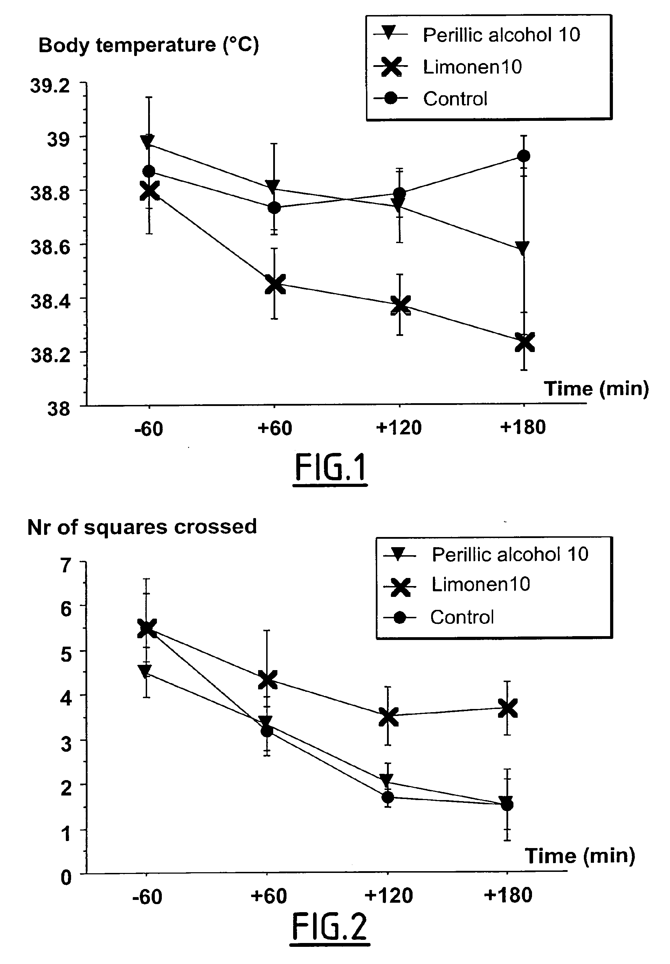

### Body temperature(see Figure 1)

The Mann-Whitney test shows that the rats of the Limonene 10 group have a body temperature significantly lower than those of the control group.

### Number of squares crossed (see Figure 2)

The Mann-Whitney test shows that the rats of the Limonene 10 group cross significantly more squares than those of the control group.

### Interest for an object (see Figure 6)

The Mann-Whitney test shows that the rats of the Limonene 10 group have an interest for an object significantly higher than those of the control group.

### Wire manoeuvre (see Figure 8)

The Mann-Whitney test shows that the rats of the Limonene 10 group manoeuvre on the wire significantly more often than those of the control group.

### Audible vocalization (see Figure 14)

The Mann-Whitney test shows that the rats of the Limonene 10 group emit significantly less vocalization than those of the control group.
The Mann-Whitney test shows that the rats of the Perillic alcohol 10 group have a tendency to emit less vocalization than those of the control group.

### Startles (see Figure 15)

The Mann-Whitney test shows that the rats of the Limonene 10 group have significantly less startles than those of the control group.

**Table 4 FOB Results 180 minutes after treatment (T+180) Kruskal-Wallis test and Mann-Whitney test vs. Control**

| **Groups** | **Control** | **Limonene 10** | **Perillic alcohol 10** |
|---|---|---|---|
| **Variables** | **(n = 6)** | **(n = 6)** | **(n = 6)** |
| **Body temperature** | | **<** | N.S. |
| | | **p < 0.01** | |
| **Number of squares crossed** | | **>** | N.S. |
| | | **p < 0.05** | |
| **Interest for an object** | | **>** | N.S. |
| | | **p < 0.05** | |
| **Wire manoeuver** | | **>** | N.S. |
| | | **p < 0.01** | |
| **Audible vocalization** | | **<** | **<** |
| | | **p<0.001** | **p<0.10** |
| **Startles** | | **<** | N.S. |
| | | **p < 0.01** | |

| | | | |
|---|---|---|---|
| Legend of Table 4 : ">" means a reflex higher than the control group "<" means a reflex lower than the control group boldface symbols mean a significant effect "N.S." = no difference with the control group | | | |

### REFERENCES

- Balcombe JP et al., Laboratory routines cause animal stress, Contemp Top Lab Anim Sci. 2004 Nov;43(6):42-51);
- Zimecki M et al., Effects of lactoferrin on the immune response modified by the immobilization stress. Pharmacol Rep. 2005 Nov-Dec;57(6):811-7;
- Zimecki M and Artym J, 2004; [The effect of psychic stress on the immune response] Postepy Hig Med Dosw (Online). Review. Polish 2004 Mar 24;58:166-75.
- Crowell PL et al., "Human metabolism of the experimental cancer therapeutic agent d-limonene", Cancer Chemother Pharmacol. 1994;35(1):31-7;
- Hardcastle IR et al., "Inhibition of protein prenylation by metabolites of limonene", Biochem Pharmacol. 1999 Apr 1;57(7):801-9;
- Vigushin DM et al., « Phase I and pharmacokinetic study of D-limonene in patients with advanced cancer. Cancer Research Campaign Phase I/II Clinical Trials Committee", Cancer Chemother Pharmacol. 1998;42(2):111-7;
- MacPhail RC, Observational batteries and motor activity. Zentralbl Bakteriol Mikrobiol Hyg [B]. 1987 Oct;185(1-2):21-7.

## Claims

1. Use of a monoterpene for the prevention or alleviation of a stress condition of an individual.

2. Use of a monoterpene for the manufacture of a medicament for the treatment of a pathological stress condition of an individual.

3. The use according to claim 1, said stress condition being a non-pathological stress condition.

4. The use according to any one of claims 1 to 3, said individual being selected in the group consisting of a human, a pet, a laboratory animal or a farm animal.

5. The use according to claim 4, said individual being a cat or a dog.

6. The use according to any one of claims 1 to 5, said monoterpene being limonene, one of its metabolites or a mixture thereof.

7. The use according to claim 6, said limonene metabolite being perillic alcohol or perillic acid.

8. The use according to claim 3, wherein said non-pathological stress condition is due to force-feeding in ducks or geese.

9. The use according to claim 3, wherein said non-pathological stress condition is due to carriage in horses.

10. The use according to claim 3, wherein said non-pathological stress condition is due to an exam in human.
